# EUROPÄISCHE PATENTSCHRIFT

(11) **EP 2 175 829 B1**
(45) Veröffentlichungstag und Bekanntmachung des Hinweises auf die Patenterteilung: **01.07.2015**
(21) Anmeldenummer: 08784643.2
(22) Anmeldetag: 08.07.2008
(51) Int. Cl.: A61K 8/34, A61Q 15/00

(54) **VERWENDUNG VON KURZKETTIGEN VICINALEN DIOLEN ALS ANTITRANSPIRANTWIRKSAME MITTEL**
USE OF SHORT-CHAIN VICINAL DIOLS AS AGENTS HAVING ANTIPERSPIRANT ACTIVITY
UTILISATION DE DIOLS VICINAUX À CHAÎNE COURTE COMME MOYENS ANTITRANSPIRANTS

(30) Priorität: 11.07.2007 DE 102007032642
(43) Veröffentlichungstag der Anmeldung: 21.04.2010
(73) Patentinhaber: Beiersdorf AG, 20253 Hamburg (DE)
(72) Erfinder: BIEL, Stefan, 21077 Hamburg (DE); GLÄSER, Katja, 22607 Hamburg (DE); THIELECKE, Ina, 22529 Hamburg (DE); TERSTEGEN, Lara, 20253 Hamburg (DE); KEYHANI, Reza, 21039 Hamburg (DE)
(74) Vertreter: Hartmann, Jost
(86) Internationale Anmeldenummer: PCT/EP2008/005549
(87) Internationale Veröffentlichungsnummer: WO 2009/007089

(56) Entgegenhaltungen:
- EP-A- 1 604 642
- WO-A-03/055559
- WO-A-2008/057317
- DE-A1- 2 433 703
- GB-A- 2 113 090
- GB-A- 2 384 789
- US-A- 2 751 356
- US-A- 4 761 278
- ANONYMOUS: "Antitranspirant" INTERNET ARTICLE, [Online] 5. Dezember 2008 (2008-12-05), XP002514458 Gefunden im Internet: URL:http://de.wikipedia.org/wiki/Antitrans pirant> [gefunden am 2009-02-09]

## Beschreibung

Die Erfindung betrifft die Verwendung von kurzkettigen vicinalen Diolen, insbesondere Glykolen, als antitranspirant wirksame Mittel.

Kosmetische Formulierungen zur Verwendung als Unterarmprodukte enthalten antitranspirante und/oder desodorierende Wirkstoffe.

Die desodorienden Wirkstoffe dienen als Schutz vor unangenehmen Gerüchen durch das Schwitzen. Verschiedenen Wirkprinzipen können den deodorierenden Wirkstoffen zu Grunde liegen. Denkbare Wirkprinzipen sind die Überdeckung des Schweißes z.B. mit Parfüm, die Absorption des Geruches durch z.B. 1,5-Pentandiol (WO 2007/063065 A1). Da der Schweißgeruch durch die Zersetzung humaner Körperausscheidungen durch Bakterien entsteht, werden auch keimhemmende, antimikrobielle Wirkstoffe und Enzyminhibitoren als desodoriende Wirkstoffe bezeichnet und in kosmetischen Zubereitungen eingesetzt (Domsch, A., Die kosmetischen Präparate,1990, Band II, 4. Auflage, Verlag für chem. Industrie, H. Ziolkowsky KG, Augsburg; Umbach, W., Kosmetik, 1995, 2. erweiterte Auflage, George Thieme Verlag, Stuttgart, New York).

Als Antitranspirantien werden Wirkstoffe bezeichnet, die das Austreten des Schweißes an die Oberfläche behindern. Dieses kann durch die Verschließung des Schweißdrüsenausführganges z.B. durch Filmbildner oder astringierende Wirkstoffe geschehen. Des Weiteren werden auch Substanzen, die die Aktivität der ekkrinen Schweißdrüse beeinflussen, als antittranspirante Wirkstoffe verwendet. (Domsch, A., Die kosmetischen Präparate,1990, Band II, 4. Auflage, Verlag für chem. Industrie, H. Ziolkowsky KG, Augsburg; Umbach, W., Kosmetik, 1995, 2. erweiterte Auflage, George Thieme Verlag, Stuttgart, New York).

Auch die Kombination von Adstringentien mit antimikrobiell wirksamen Stoffen in ein und derselben Zusammensetzung ist gebräuchlich.

Es ist damit klar zwischen deodorierend und antitranspirant wirkend zu unterscheiden.

Heutzutage beinhalten nahezu alle kosmetischen Antitranspirantien Astringentien- vorwiegend Aluminiumsalz wie das Aluminiumhydroxychlorid (Aluminiumchlorhydrat, ACH oder aktiviertes ACH-AACH) oder Aluminium-Zirkonium-Salz (AZG) als antitranspiranten Wirkstoff.

Diese auf Aluminiumsalzen basierenden AT-Mittel reduzieren zwar die vom Körper abgegebene Schweißmenge, führen aber als ungewollter Nebeneffekt zu Hautreizungen sowie zu weißen Rückständen auf der Haut oder Kleidung nach dem Eintrocknen bzw. bei Langzeitanwendung zu Ablagerungen in der Kleidung. Außerdem führen der niedrige pH-Wert (< 5) und die Elektrolyteigenschaften der als Antitranspirant einsetzbaren Aluminiumsalze zu Einschränkungen bei der Herstellung kosmetischer Formulierungen für die Anwendung auf der Haut, sowohl hinsichtlich der Rohstoffauswahl (Säureverträglichkeit; keine starken Basen, da diese zur Ausfällung von Al(OH)₃ führen) als auch hinsichtlich der Lagerstabilität.

Wünschenswert wäre es demnach ein Antitranspirant zur Verfügung zu stellen, das die vorgenannten Nachteile und Nebenwirkungen nicht aufzeigt.

Wünschenswert wäre es weiterhin eine Antitranspirantzubereitung zur Verfügung zu stellen, die den Stand der Technik bereichert und eine Alternative zu den bekannten Zubereitungen darstellt.

Insbesondere ist es wünschenswert eine kosmetische und/oder dermatologische Antitranspirant-Formulierung mit einer Antitranspirantwirkung bereitzustellen ohne die Nachteile aluminiumhaltiger Zubereitungen aufzuweisen.

Insbesondere ist es wünschenswert eine solche Zubereitung zur Verfügung zu stellen, die zur Stabilisierung und/oder Aufrechterhaltung der Antitranspirantwirkung eines Antitranspirants nach dem Schwitzen und/oder Hautreinigung beiträgt.

Wünschenswert wäre zudem ein Antitranspirant, das im Gegensatz zu ACH-haltigen Systemen zusätzlich zur schweißhemmenden Wirkung eine Hautbefeuchtung der Achselnaut ermöglicht, da ACH-haltige Zubereitungen häufig ein Austrocknung und damit verbundenen zusätzlichen Reizung der Haut verursachen.

Insbesondere ist es auch wünschenswert einen Antitranspirantwirkstoff zur Verfügung zu stellen, der möglichst breite Möglichkeiten der galenischen Einarbeitung in kosmetisch akzeptable und attraktive Formelsysteme ermöglicht.

Bekannt ist, dass Glykole als Formelbestandteile in kosmetischen Zubereitungen und u.a. auch in Kombination mit ACH eingesetzt werden, wie beispielsweise in EP 1206 239 B1.

US 7235229 B2, US 2007/0202062 oder EP 1 206 237 B1 beschrieben.

In EP 1478 231 B1 und DE 199 24 496 A1 werden längerkettige Diole (C5 -C10) als antimikrobiell wirksame, also desodoriende Stoffe beschrieben und deren Einsatz als Deodorant und Konservierungsmittelhelfer. Des Weiteren werden Diole als Geruchsreduzierer und Absorber in Sanitärartikeln und Sprays eingesetzt (WO 2007/063065).

Eine schweißhemmende, antitranspirant Wirkung der Diole wird im Stand der Technik nicht festgestellt. Vielmehr wird deren Kombination mit schweißhemmenden Wirkstoffen (Antitranspirantien) als bevorzugt offenbart.

Überraschend wurde nun herausgefunden, dass kurzkettige vicinale, kurzkettige Glykole (C2 bis C4), vor allem 1,2-Propylenglykol (auch 1,2-Propandiol) und 2,3-Butylenglykol (2,3-Butylenglykol) sowie Ethylenglykol, eine schweißhemmende Wirkung zeigen.

Die Erfindung umfasst demnach die Verwendung von vicinalen kurzkettigen Diolen mit einer C-Atomanzahl von 2 bis 4 vor allem 1,2- Ethylenglykol, 1,2-Propylenglykol, 1,2 Butylenglykol und/oder 2,3-Butylenglykol, als Antitranspirantwirkstoff, in topisch applizierbaren, insbesondere kosmetischen und/oder dermatologischen, Zubereitungen wobei der Anteil an vicinalen Diolen im Bereich von 20 bis 50 GW.-%, bezogen auf die Gesamtmasse der Zubereitung, gehählt wird.

Glykol ist eine von der Stammverbindung Ethylenglycol abgeleitete Gattungsbezeichnung für Diole, deren Hydroxy-Gruppen *vicinal* angeordnet sind und die daher auch *vic*-Diole oder 1,2-Diole genannt werden.

Zu den erfindungsgemäßen kurzkettigen vicinalen Diolen zählen Diole mit einer C-Atomanzahl von 2 bis 4, wie insbesondere die Glykole Ethylenglykol (Monoethylenglycol, Diethylenglycol), Propylenglykol (Propan-1,2-diol), Butylenglykol (1,3-Butylen-1,2-glycol und Butylen-2,3-glykol) als auch deren Mischungen. Es sei darauf hingewiesen, dass der Begriff 1,2-Diol im Rahmen des vorliegenden Textes sowohl das entsprechende 2S-konfigurierte Enantiomer als auch das 2R-konfigurierte Enantiomer sowie beliebige Mischungen aus diesen 2S- und 2R-konfigurierten Enantionmeren umfasst. Im Falle des 2,3-Butandiols tritt neben der S,S-(L)-Konfiguration und der R,R-(D)-Konfiguration eine R,S (bzw.S,R)- (meso)-Konfiguration auf, die erfindungsgemäß ebenfalls umfasst ist.

Aus kommerziellen Gründen ist es zwar besonders vorteilhaft, Gemische von auf beiden Enantiomeren der jeweiligen Diole als antitranspiranten Wirkstoff einzusetzen, da diese synthetisch besonders leicht zugänglich sind und keiner weiteren Aufreinigung bedürfen.

Die Erfindung umfasst sowohl die reinen Enantiomere als auch Enantiomer-Gemische. Bevorzugt sind darüber hinaus 1,2-Diole als R und S- Konfiguration sowie 2,3-Butylenglykol in der D- und L- Konfiguration.

1,2-Propylenglycol ist in Deutschland als Zusatzstoff nur als Lösemittel für Antioxidantien, Emulgatoren und Enzyme (ZZuIV) sowie als Lösemittel für Aromen zugelassen [Aromen-Verordnung § 3 (Nr. 3), Anl. 2]. Die Verwendung in Kosmetika unterliegt, sofern die Kosmetik-Verordnung nicht berührt wird, keiner rechtlichen Beschränkung.

Überraschenderweise zeigte sich, dass kurzkettige vicinale Diole, insbesondere Glykole, insbesondere 1,2-Propylenglykol, die bekanntermaßen nicht zu den im Stand der Technik zitierten Antitranspirantien (AT) zählen, eine antitranspirante, schweißhemmende Wirkung zeigen.

Dies ist umso mehr erstaunlich, da die AT-Leistung einer Zubereitung umfassend 30 Gew.% 1,2-Propylenglykol (analog Beispielrezeptur 13), bezogen auf die Gesamtmasse der Zubereitung, besser ist als die AT-Leistung einer Zubereitung umfassend 10 Gew.% Aluminiumchlorohydrat (siehe Abbildung a). Die Schweißreduktion wurde in Anlehnung an M. Keskin et al. J. Invest. Dermatol. 1998; 110:591 gemessen. Bei dieser Messung werden die Testsubstanzen auf dem Unterarm der Probanden angewendet. Die Probanden werden bei 75 °C zum Schwitzen angeregt; nach Beginn der Schweißproduktion wird mittels Silikonabdrucktechnik die Anzahl der schweißabgebenden Schweißdrüsen im Testareal ermittelt und gegen ein unbehandeltes Vergleichsareal abgeglichen. In den Abbildungen a ist die Restschweißmenge im Vergleich zum unbehandelten Areal nach Produktapplikation dargestellt. 100% Restschweißmenge entsprechen 0% Schweißreduktion.

D.h. die erfindungsgemäße Verwendung von kurzkettigen vicinalen Diolen ermöglicht eine bessere schweißhemmende Wirkung als bekannte und bewährte Zubereitung mit antitranspirantem Wirkstoff (mit ACH).

Abbildung b zeigt, dass erfindungsgemäße vicinale Diole den Schweißfluss signifikant reduzieren, hingegen nicht erfindungsgemäße Diole nicht. Die schweißhemmende Wirkung wurde wie für Abbildung a beschrieben getestet.

Die Einsatzkonzentration der in Abbildung b gegenübergestellten Zubereitungen beträgt bei den reinen Diolen 50 % in Wasser, bei ACH 10%. Es ist die mittlere Restaktivität der Schweißdrüsen dargestellt. Je niedriger die Restaktivität desto besser ist die antitranspirante Wirkung.

Die gemessene antitranspirante Wirkung zeigt zudem auch in kosmetischen Zubereitungen eine lineare Abhängigkeit von der Einsatzkonzentration (Beispiel 1,2-Propylenglykol in zwei unterschiedlichen Zubereitungen, siehe Abbildung c, (O/W-Makroemulsion, analog Beispielrezeptur 9; verdickte O/W-Emulsion, analog Beispielrezeptur 13). Das Testdesign ist wie für Abbildung a beschrieben. Es ist die mittlere Restaktivität der Schweißdrüsen dargestellt. Je niedriger die Restaktivität desto besser ist die antitranspirante Wirkung.

In allen drei Abbildungen (a, b und c) sieht man sehr deutlich, dass ohne bislang bekannte antitranspirante wirksame Stoffe, wie ACH, dennoch eine schweißhemmende Wirkung nur allein aufgrund der Glykole zu beobachten ist.

Abbildung d stellt die schweißhemmende Wirkung einer Zubereitung umfassend 45 Gew.% Ethylenglykol im Vergleich zu wässrigen Lösung umfassend 10 Gew.% ACH, gemessen aus einer O/W-Emulsion in Anlehnung an G.E. Piérard et al., Skin Pharmacol Appl Skin Physiol 2003;16:324-342 und M. Keskin et al., J. Invest. Dermatol. 1998;110:591 dar. Getestet wurde dabei ein System entsprechend Beispielrezeptur 10 (enthaltend 45% Ethylenglykol). Die schweißhemmende Wirkung wird dadurch ermittelt, dass die Testsubstanzen bei Probanden einseitig in der Achsel aufgetragen werden; die andere Achsel bleibt als Kontrollareal unbehandelt. Nach dem Schwitzen (Hotroom in Anlehnung an FDA-Richtlinien) wird die abgegebene Schweißmenge vor und nach Produktapplikation gravimetrisch an in der Achsel platzierten, schweißaufnehmenden Pads ermittelt und die Schweißmenge von behandelter und unbehandelter Achsel ins Verhältnis gesetzt.

Aufgetragen ist in Abbildung d die Restschweißmenge im Vergleich zum unbehandelten Areal nach Produktapplikation; 100% Restschweißmenge entsprechen 0% Schweißreduktion.

Die Daten belegen, dass die Schweißreduktion von 10% ACH und 45% Ethylenglykol, verabreicht unter der Achsel beispielsweise aus einer O/W Emulsion, annähernd identisch ist.

Vorteilhaft ist, dass in erfindungsgemäßen kosmetischen Zubereitungen umfassend kurzkettige vicinale Diole, vornehmlich 1,2-Propylenglykol, gänzlich auf andere Antitranspirantwirkstoffe verzichten kann und dennoch ein ausreichender Antitranspirantschutz gewährleistet bleibt.

Dies führt auch zur Behebung der aufgeführten Nachteile, wie Hautreizung und Haut- bzw. Kleidungverfärbungen (Weißein), da diese vor allem auf die Gegenwart von ACH zurück zuführen sind.

Bevorzugt umfassen erfindungsgemäße kosmetische Zubereitung daher neben den kurzkettigen vicinalen Diolen keine weiteren astringierende, antitranspirant wirksamen Stoffe, insbesondere keine Aluminiumsalze, insbesondere kein ACH und/oder AACH (aktiviertes Aluminiumchlorohydrat).

Eine Kombination aus den erfindungsgemäßen Diolen mit schweißreduzierenden Mitteln, die nicht auf einem astringierenden Wirkmechanismus beruhen, bevorzugt AT-mittel aus der Gruppen der Anticholinergika, wie beispielsweise 4-[(2-Cyclopentyl-2-hydroxyphenylacetyl)oxy]-1,1-dimethyl-piperidiniumsalze, insbesondere das 4-[(2-Cyclopentyl-2-hydroxyphenylacetyl)oxy]-1,1-dimethyl-piperidiniumbromid (Glycopiperolat) können zu einem Anteil von bevorzugt 0,05 bis1,0 Gew.%, vorzugsweise 0,1%-0,7%, insbesondere 0,3%-0,5 Gew.%, bezogen auf die Gesamtmasse der Zubereitung zugesetzt werden. Eine Verstärkung der antitranspiranten Wirkung ist damit erreichbar.

Bekannt ist, dass Zubereitungen mit Al-Salzen eine relativ dauerhafte Verstopfung der Schweißpore herbeiführen und daher auch eine AT-Wirksamkeit über mehrere Stunden bzw. Tage aufweisen können. D.h., dass auch am Tag nach der Applikation noch eine Wirkung vorhanden sein kann. Nachteilig ist aber, wie erläutert, dass es dabei zu Hautirritationen kommen kann.

Die kurzkettigen Glykole, insbesondere 1,2-Propylenglykol, zeigen nun erstaunlicherweise eine unmittelbare AT-Wirkung (Sofort-Effekt), die bereits nach dem ersten Auftragen messbar ist und nicht durch mehrfache Applikation aufgebaut werden muss, jedoch ohne den Hautreizungen fördernden Depot-Effekt der Aluminiumsalze.

Wesentlicher Vorteil der erfindungsgemäßen Zubereitungen ist darüber hinaus, dass sich gegenüber den auf Aluminiumsalzen basierenden AT-Mitteln, keinerlei Verfärbungen auf der Haut oder Kleidung zeigt. Das sogenannte Weißeln unterbleibt ebenso wie die nach mehrfachem Tragen und Waschen in direkt auf der Achselhaut aufliegenden Textilien zu beobachtenden Rückstände. Ein weiterer Vorteil von antitranspiranten Zubereitungen, die Glykole als schweißhemmendes Mittel enthalten, ist die zusätzliche Pflege und damit verbunden Schutz der Haut.

Glykole werden in kosmetischen Zubereitungen bereits als Moisturizer eingesetzt und wirken in den erfindungsgemäßen Zubereitungen sodann zweifach, als Hautpflegender Wirkstoff und als Antitranspirantwirker.

Als Antitranspirantwirkung wird die Möglichkeit der Verminderung oder Verhinderung der Schweißbildung verstanden. D.h. Erfindungsgemäße kurzkettige Glykole (vicinale Diole) wirken als Schweißhemmer, vermindern also die Schweißbildung.

Die erfindungsgemäßen Zubereitungen reduzieren den Schweißfluss (gemessen in Anlehung an G.E. Piérard et al., Skin Pharmacol Appl Skin Physiol 2003;16:324-342 und M. Keskin et al., J. Invest. Dermatol. 1998;110:591.) schon in geringen Konzentration von etwa 15 % um mehr als 40%, im Vergleich zum Schweißfluss ohne Auftrag der Zubereitung (siehe Abbildung c). Selbst nach starkem Schwitzen oder Körperreinigung ist noch eine etwas verminderte Schweißfluss-Reduktion zu beobachten, ohne dass vorher erneut Produkt aufgetragen wurde.

Kurzkettige 1,2 - Diole, insbesondere 1,2-Propylenglykol, auch als 1,2-Propandiol bezeichnet, sind als Lösungsmittel auch in kosmetischen Zubereitungen bekannt. Eine Verwendung in AT- bzw. Deodorantien als AT-Wirkstoff ist jedoch nicht bekannt.

Vorteilhaft können erfindungsgemäßen Zubereitungen auch Desodorantien zugesetzt werden.

Alle für Desodorantien gängigen Wirkstoffe können vorteilhaft zusätzlich genutzt werden, beispielsweise Geruchsüberdecker wie die gängigen Parfümbestandteile, Geruchsabsorber, beispielsweise die in der DE 40 09 347 beschriebenen Schichtsilikate, von diesen insbesondere Montmorillonit, Kaolinit, Ilit, Beidellit, Nontronit, Saponit, Hectorit, Bentonit, Smectit, ferner beispielsweise Zinksalze der Ricinolsäure. Keimhemmende Mittel sind ebenfalls geeignet, in die erfindungsgemäßen Zubereitungen eingearbeitet zu werden. Vorteilhafte Substanzen sind zum Beispiel 2,4,4'-Trichlor-2'-hdroxydiphenylether (Irgasan), 1,6-Di-(4-chlorphenylbiguanido)-hexan (Chlorhexidin), 3,4,4'-Trichlorcarbanilid, quaternäre Ammoniumverbindungen, Nelkenöl, Minzöl, Thymianöl, Triethylcitrat, Famesol (3,7,11-Trimethyl-2,6,10-dodecatrien-1-ol) sowie die in den DE 37 40 186, DE 39 38 140, DE 42 04 321, DE 42 29 707, DE 42 29 737, DE 42 37 081, DE 43 09 372, DE 43 24 219 beschriebenen wirksamen Agenzien. Auch Natriumhydrogencarbonat ist vorteilhaft zu verwenden. Als deosodierende Wirkstoffe können in kosmetischen Zubereitungen auch geringe Mengen an Adstringenzien, z.B. Alaune eingesetzt werden. Die keimhemmende Wirkung von geringen Mengen an adstringierenden Substanzen wurde bereits im Patent EP 96 02108 beschrieben. Vorteilhaft sind dabei Konzentration zwischen 1 % und 8 %.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, UV-Filter, Antioxidantien, wasserlösliche Vitamine, Mineralstoffe, suspendierte Festkörperpartikel, Parfüme, Substanzen zum Verhindern des Schäumens, Farbstoffe, Pigmente, die eine färbende Wirkung haben, Verdickungsmittel, anfeuchtende und/oder feuchthaltende Substanzen oder andere übliche Bestandteile einer kosmetischen oder dermatologischen Formulierung wie Alkohole, Polyole, Polymere, Schaumstabilisatoren oder Silikonderivate.

Bevorzugt ist die Kombination mit Detergenzien, wie z.B. Konserveriungsmittel, Ethanol oder Parfümkompositionen, die zu einer Verstärkung der antitranspiranten Wirksamkeit der Diolen führen. Die Einsatzkonzentration von Ethanol liegt dabei zwischen 0 und 50 % bevorzugt zwischen 10 und 50 %. Es konnte dabei ein Einfluss auf die AT-Wirksamkeit der Diole festgestellt werden. Beispielweise zeigen wässrigen Lösungen enthaltend 35 % 2,3-Butylenglykol mit 32.5 % Ethanol ein bessere antitranspirante Wirkung als in rein wässriger bzw. rein alkoholischer Lösung (siehe Abbildung e). Die schweißreduzierende Wirkung wurde wie für Abbildung a (siehe S. 5) beschrieben ermittelt. Dargestellt ist die Restaktivität der Schweißdrüsen im Vergleich zum unbehandelten Areal nach Produktapplikation. 100% Restaktivität bedeuten 0 % Schweißreduktion.

Die erfindungsgemäße kosmetische Zubereitung ist vorteilhaft dadurch gekennzeichnet, das sie in Form einer wässrigen oder wässrig-alkoholischen Lösung, einer Emulsion (W/O, O/W, W/Si, Si/W oder multiple Emulsion, Makro-, Mikro- oder Nanoemulsion), einer Dispersion, einer Pickering-Emulsion, eines Gels, eines Hydrodispersionsgels oder einer wasserfreien Zubereitung vorliegt.

Die Zubereitung kann erfindungsgemäß auch in Form einer dünnflüssigen, sprühfähigen wässrigen oder wässrig-alkoholischen Lösung, in Form eines Gels, in einer Wachsmatrix, einer Stiftform, als Salbe, Creme oder Lotion (ggf. sprühbar) vorliegen.

Die Zubereitung kann erfindungsgemäß vorteilhaft auch als Aerosol, Spray oder als Tränkungsmedium für ein Pflaster oder Tuch eingesetzt werden. Daher sind auch Pflaster und Tücher getränkt mit der erfindungsgemäßen Zubereitung, erfindungsgemäß.

Als kosmetische Zubereitung werden die erfindungsgemäßen Diole bevorzugt formuliert als Gel, Stift, Aerosol und/oder als Emulsion. Als Gel wird der Vorteil generiert, dass auch Gelbildner verwendet werden können, die ansonsten in Kombination mit ACH nicht verwendet werden können. Als Stift oder Aerosol bietet sich der Vorteil der Vermeidung von weißen Rückständen auf Haut und Kleidung. Als Emulsion formuliert bietet die erfindungsgemäße Zubereitung gegenüber ACH-haltigen Formulierungen ein besseres Hautgefühl und ermöglicht eine bessere Hautbefeuchtung.

Zur Applikation der Zubereitung lassen sich vorteilhaft herkömmliche Packmittel für kosmetische Zubereitungen, insbesondere Deodorantien und/oder Antitranspirantien verwenden, z. B. Aerosole, Zerstäuber, Stiftdispenser, Geldispenser, Tuben und Roller.

### Beispielrezepturen

### Angaben in Gewichtsprozent bezogen auf die Gesamtmasse der Zubereitungen

### Beispiel 1 - 8: Transparente Gelsysteme

| **Rohstoff** | **Beispiel 1** | **Beispiel 2** | **Beispiel 3** | **Beispiel 4** |
|---|---|---|---|---|
| Ethylenglykol | 20,0% | | | 10,0% |
| 1,2-Propylenglykol | | 25,00% | | 10,0% |
| 2,3-Butylenglykol (D/L-Isomere) | | | 20,00% | |
| Glycopiperolat | | | 0,50% | |
| PEG-40 Hydrogenated Castor Oil | 5,00% | 5,50% | 4,30% | 5,00% |
| Cetyl Hydroxyethylcellulose | 1,50% | 1,00% | 0,90% | 1,50% |
| Keimhemmende Mittel/ Deosodierender Wirkstoff | q.s. | q.s. | q.s. | q.s. |
| Parfüm, Antioxidantien | q.s. | q.s. | q.s. | q.s. |
| Wasser | ad 100% | ad 100% | ad 100% | ad 100% |
| | | | | |
| **Rohstoff** | **Beispiel 5** | **Beispiel 6** | **Beispiel 7** | **Beispiel 8** |
| Ethylenglykol | 45,00% | | 10,00% | 40,00% |
| 1,2-Propylenglykol | | 35,00% | | |
| 2,3-Butylenglykol (D/L-Isomere) | | | 20,00% | |
| Glycopiperolat | | 0,50% | | |
| PEG-8 | 2,00% | 1,80% | 1,50% | 1,50% |
| PEG-40 Hydrogenated Castor Oil | 2,00% | 1,80% | 1,50% | 1,50% |
| Hydroxyethylcellulose | 0,35% | 0,50% | 0,30% | |
| Octyldodecanol | 0,10% | 0,15% | 0,08% | 0,08% |
| Zitronensäure | | 0,03% | | |
| Parfüm, Antioxidantien | q.s. | q.s. | q.s. | q.s. |
| Wasser | ad 100% | ad 100% | ad 100% | ad 100% |

### Beispiel 9 - 12: O/W Emulsion

| **Rohstoff** | **Beispiel 9** | **Beispiel 10** | **Beispiel 11** | **Beispiel 12** |
|---|---|---|---|---|
| Ethylenglykol | | 45,00% | | |
| 1,2-Propylenglykol | 20,00% | | | 5,00% |
| 1,2-Butylenglykol | | | | 10,00% |
| 2,3-Butylenglykol (D/L-Isomere) | | | 30,00% | |
| Glycopiperolat | | | 0,50% | |
| Polyethylenglykol(21)stearylether | 2,00% | 1,90% | 2,00% | 2,00% |
| Polyethylenglykol(2)stearylether | 2,50% | 2,60% | 2,50% | 2,40% |
| Polypropylenglykol(15)stearylether | 3,00% | 3,10% | 3,00% | 2,80% |
| EDTA | 1,50% | 1,40% | 1,50% | 1,30% |
| Parfüm, Antioxidantien | q.s. | q.s. | q.s. | q.s. |
| Wasser | ad 100% | ad 100% | ad 100% | ad 100% |

pH-Werte einstellen mit Natronlauge/Zitronensäure auf pH 5,5

### Beispiel 13 - 15: O/W Emulsion

| **Rohstoff** | **Beispiel 13** | **Beispiel 14** | **Beispiel 15** |
|---|---|---|---|
| 1,2-Propylene Glycol | 30,00% | | |
| 1,2-Butylenglykol | | | 15,00% |
| 2,3-Butylenglykol | | 20,00% | |
| Glycpiperolat | | | 0,50% |
| Dimethicone | 1,00% | | 2,00% |
| Alcohol Denat. | 5,00% | 10,00% | |
| Acrylates/C10-30 Alkyl Acrylate Crosspolymer | 0,10% | 0,05% | 0,15% |
| Acrylates/C10-30 Alkyl Acrylate Crosspolymer | 0,20% | 0,10% | 0,30% |
| Cyclomethicone | 10,00% | 15,00% | 10,00% |
| Sodium Stearoyl Glutamate | 0,20% | 0,20% | 0,20% |
| Sucrose Polystearate + Hydrogenated Polyisobutene | 1,00% | 1,00% | 1,00% |
| Keimhemmendes Mittel/ Deosodierender Wirkstoff | q.s. | q.s. | q.s. |
| Parfüm, Antioxidant | q.s. | q.s. | q.s. |
| Wasser | ad 100% | ad 100% | ad 100% |

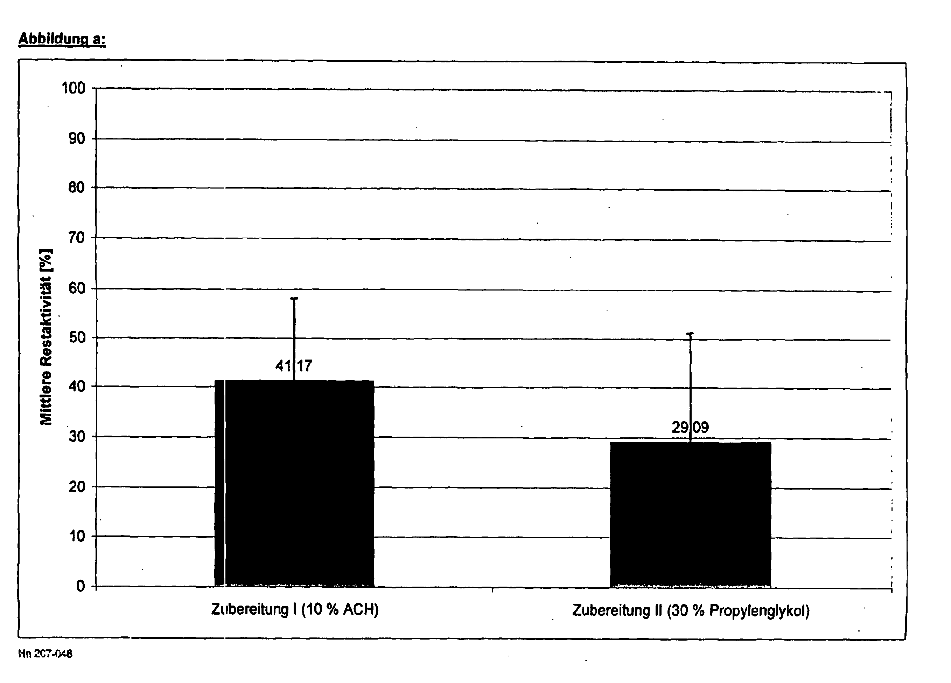

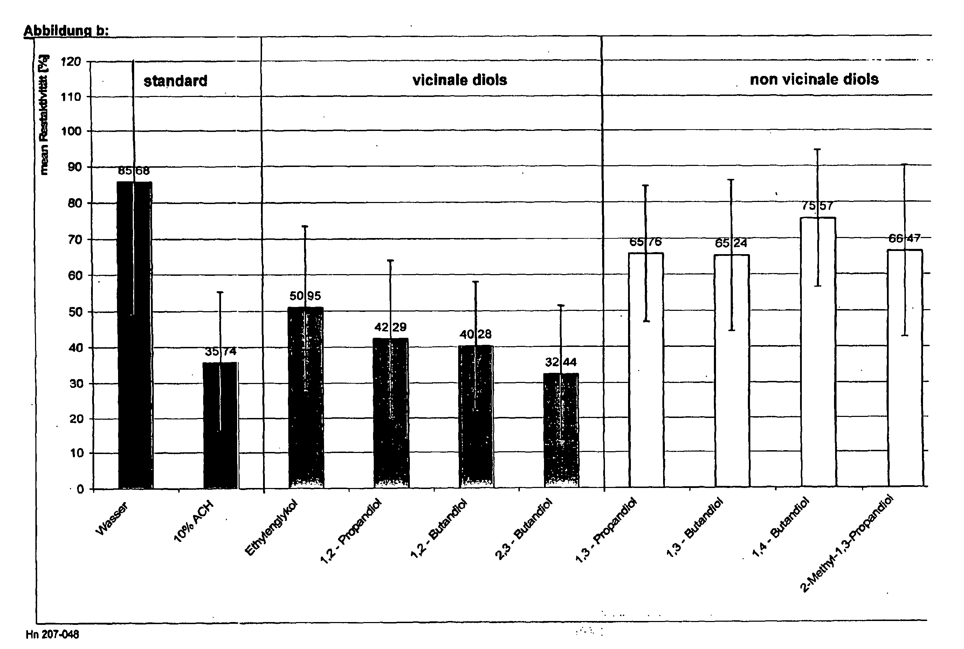

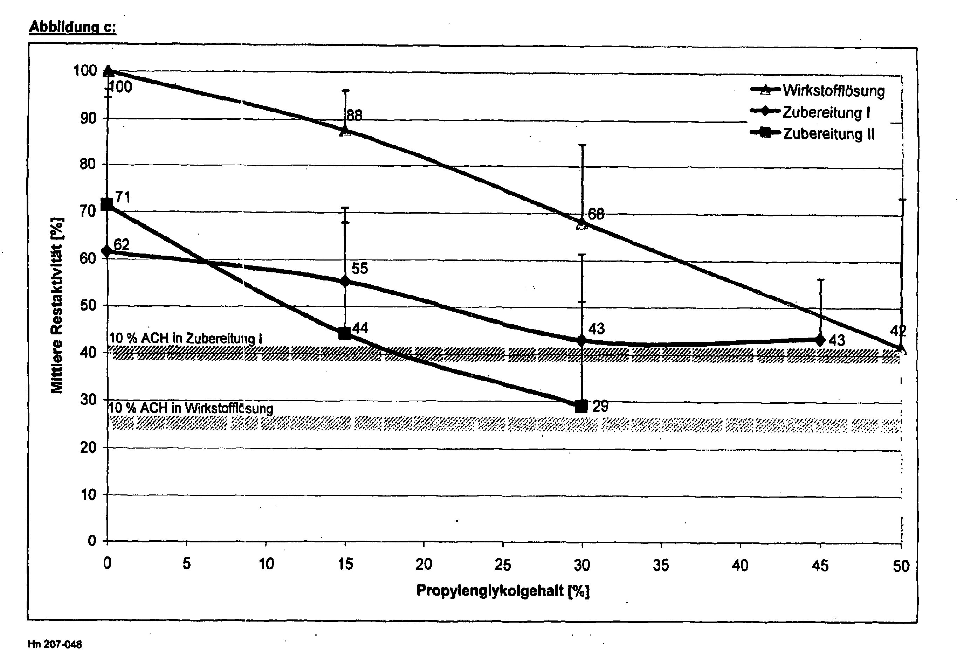

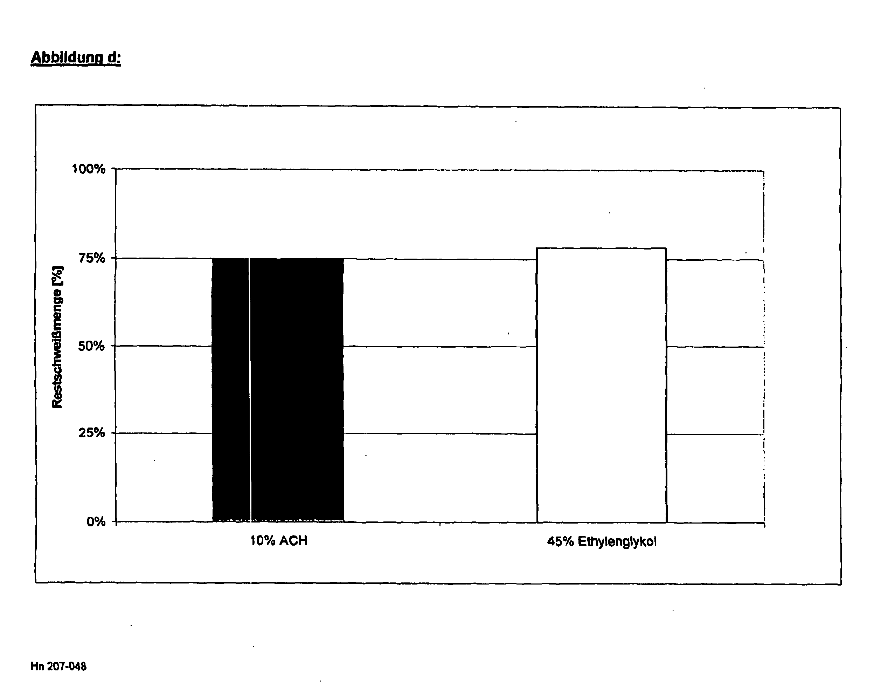

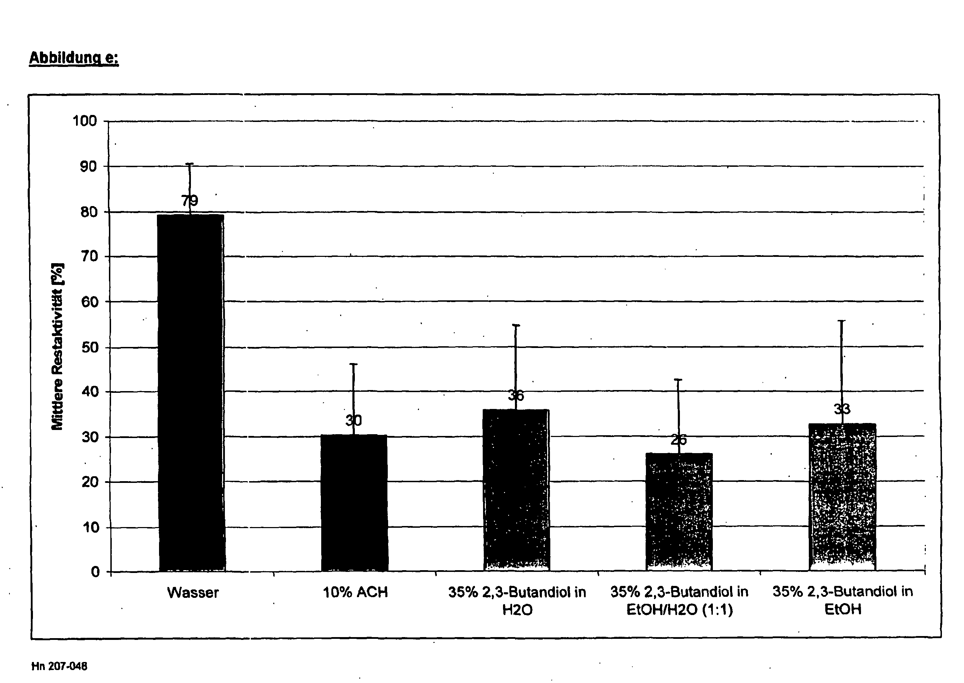

## Patentansprüche

1. Verwendung von kurzkettigen vicinalen Diolen mit einer C-Atomanzahl von 2 bis 4 als antitranspirant- und/oder schweißhemmenden Wirkstoff in topisch applizierbaren Zubereitungen, **dadurch gekennzeichnet, dass** der Anteil an vicinalen Diolen im Bereich von 20 bis 50 Gew.-%, bezogen auf die Gesamtmasse der Zubereitung, gewählt wird.

2. Verwendung nach Anspruch 1, **dadurch gekennzeichnet, dass** Ethylenglycol, 1,2-Propylenglykol und/oder 2,3-Butylenglykol, insbesondere D und L- 2,3-Butylenglykol, als antitranspirant- und/oder schweißhemmenden Wirkstoff gewählt wird.

3. Verwendung nach Anspruch 1 oder 2, **dadurch gekennzeichnet, dass** die Diole in topisch applizierbaren kosmetischen Zubereitungen enthalten sind.

4. Verwendung nach Anspruch 3, **dadurch gekennzeichnet, dass** zusätzlich Ethanol zugesetzt wird.

5. Verwendung von vicinalen Diolen mit einer C-Atomanzahl von 2 bis 4 nach einem der vorstehenden Ansprüche als alleiniger Antitranspirantwirkstoff in kosmetischen Zubereitungen.

## Claims

1. Use of short-chain vicinal diols having a carbon atom number of 2 to 4 as antiperspirant and/or perspiration-inhibiting active ingredient in preparations for topical application, **characterized in that** the proportion of vicinal diols is selected in the range of 20 to 50% by weight, based on the total mass of the preparation.

2. Use according to Claim 1, **characterized in that** the antiperspirant and/or perspiration-inhibiting active ingredient selected is ethylene glycol, 1,2-propylene glycol and/or 2,3-butylene glycol, in particular D- and L-2,3-butylene glycol.

3. Use according to Claim 1 or 2, **characterized in that** the diols are present in cosmetic preparations for topical application.

4. Use according to Claim 3, **characterized in that** in addition ethanol is added.

5. Use of vicinal diols having a carbon atom number of 2 to 4 according to any of the preceding claims as sole antiperspirant active ingredient in cosmetic preparations.

## Revendications

1. Utilisation de diols vicinaux à chaîne courte ayant un nombre d'atomes C de 2 à 4 en tant qu'agent actif anti-transpirant dans des préparations applicables topiquement, **caractérisée en ce que** la proportion de diols vicinaux est choisie dans la plage allant de 20 à 50 % en poids, par rapport à la masse totale de la préparation.

2. Utilisation selon la revendication 1, **caractérisée en ce que** l'éthylène glycol, le 1,2-propylène glycol et/ou le 2,3-butylène glycol, notamment le D- et le L-2,3-butylène glycol, est choisi en tant qu'agent actif anti-transpirant.

3. Utilisation selon la revendication 1 ou 2, **caractérisée en ce que** les diols sont contenus dans des préparations cosmétiques applicables topiquement.

4. Utilisation selon la revendication 3, **caractérisée en ce que** de l'éthanol est en outre ajouté.

5. Utilisation de diols vicinaux ayant un nombre d'atomes C de 2 à 4 selon l'une quelconque des revendications précédentes en tant qu'unique agent actif anti-transpirant dans des préparations cosmétiques.
